# EUROPEAN PATENT SPECIFICATION

(11) **EP 1 401 438 B1**
(45) Date of publication and mention of the grant of the patent: **28.09.2005**
(21) Application number: 01952097.2
(22) Date of filing: 02.07.2001
(51) Int. Cl.: A61K 31/4355, A61P 35/00

(54) **USE OF CYCLOPAMINE IN THE TREATMENT OF BASAL CELL CARCINOMA AND OTHER TUMORS**
VERWENDUNG VON CYCLOPAMINE, ZUR BEHANDLUNG DES BASALZELLKARZINOMS UND ANDERER TUMOREN
UTILISATION DE CYCLOPAMINE DANS LE TRAITEMENT DE CARCINOME BASO-CELLULAIRE ET D'AUTRES TUMEURS

(43) Date of publication of application: 31.03.2004
(60) Divisional application: 05008758.4
(73) Proprietor: Tas, Sinan, Izmir, 35320 (TR)
(72) Inventor: Dr. Sinan Tas, Izmir (TR); Dr.Oktay Avci, C Blok 5-1/12, Bornova, Izmir (TR)
(74) Representative: Glas, Holger
(86) International application number: PCT/TR2001/000027
(87) International publication number: WO 2002/078703

(56) References cited:
- WO-A-00/41545
- WO-A-01/27135
- WO-A-99/52534
- TAIPALE ET AL: "Effects of oncogenic mutations in Smoothened and Patched can be reversed by cyclopamine" NATURE, MACMILLAN JOURNALS LTD. LONDON, GB, vol. 406, no. 6799, 31 August 2000 (2000-08-31), pages 1005-1009, XP002166408 ISSN: 0028-0836
- DETMER K ET AL: "Erythroid differentiation in vitro is blocked by cyclopamine, an inhibitor of hedgehog signaling." DEVELOPMENTAL BIOLOGY, vol. 222, no. 1, 1 June 2000 (2000-06-01), page 242 XP008001023 Fifty-ninth Annual Meeting of the Society for Developmental Biology;Boulder, Colorado, USA; June 07-11, 2000 ISSN: 0012-1606
- HOPYAN SEVAN ET AL: "Indian Hedgehog and Parathyroid hormone related protein signaling in cartilage tumors of bone." PROCEEDINGS OF THE AMERICAN ASSOCIATION FOR CANCER RESEARCH ANNUAL, no. 41, March 2000 (2000-03), page 206 XP008001019 91st Annual Meeting of the American Association for Cancer Research.;San Francisco, California, USA; April 01-05, 2000, March, 2000 ISSN: 0197-016X
- BERMAN DAVID M ET AL: "Inhibition of prostate morphogenesis by the Sonic hedgehog pathway inhibitor cyclopamine." JOURNAL OF UROLOGY, vol. 163, no. 4 Suppl., April 2000 (2000-04), page 204 XP008001018 95th Annual Meeting of the American Urological Association, Inc.;Atlanta, Georgia, USA; April 29, 2000-May 04, 1999 ISSN: 0022-5347

## Description

This invention concerns the use of cyclopamine in vivo on basal cell carcinomas (BCC's) to achieve therapeutic effect by causing differentiation of the tumor cells and, at the same time, apoptotic death and removal of these tumor cells while preserving the normal tissue cells, including the undifferentiated cells of the normal epidermal basal layer and hair follicles. Causation of apoptosis by cyclopamine is by a non-genotoxic mechanism and thus unlike the radiation therapy and most of the currently used cancer chemotherapeutics which act by causing DNA-damage. These novel effects, previously unachieved by a cancer chemotherapeutic, make the use of cyclopamine highly desirable in cancer therapy, in the treatment of BCC's and other tumors that use the *hedgehog*/*smoothened* signal transduction pathway for proliferation and prevention of apoptosis.

Basal cell carcinoma is a common epithelial tumor. Its incidence increases with increasing age. Current treatments for BCC's include the surgical excision of the tumor together with a margin of normal tissue and, when surgery is not feasible or desirable, destruction of the tumor cells by ionizing radiation or other means. Although scarring and disfigurement are potential side effects, surgical excisions that do not leave neoplastic cells behind can provide cure. Radiation therapy acts by causing irreparably high quantity of DNA-damage which, in turn, triggers apoptotic death of the tumor cells. This mode of action of radiation-therapy, i.e. apoptosis secondary to DNA-damage, is similar to those of many chemotherapeutic agents that are currently used in the treatment of cancers. However both radiation therapy and the cytotoxic cancer chemotherapeutics are capable of causing DNA-damage in the normal cells of patients in addition to the tumor cells. As a result their effectivity and usefulness in cancer therapy are seriously limited. A further dilemma with the use of radiation and genotoxic cancer chemotherapeutics is the disturbing fact that, even when cure of the primary tumor is achieved, patients have markedly increased risk of developing new cancers because of the DNA-damage and the resulting mutations they have undergone during the treatment of primary tumor. Induction of apoptosis selectively in tumor cells by non-genotoxic means would therefore be most desirable in the field of cancer therapy.

BCC's frequently show inactivating mutations of the gene patched which encodes a transmembrane protein acting as a receptor for the hedgehog proteins identified first by their effect on the patterning of tissues during development. When not liganded by hedgehog, the patched protein acts to inhibit intracellular signal transduction by another transmembrane protein, *smoothened*. Binding of *hedgehog* to the patched causes relieving of this inhibition. Intracellular signal transduction by the relieved *smoothened* then initiates a series of cellular events resulting ultimately in alterations of the expressions of the *hedgehog* target genes and of cellular behaviour. General features of this *hedgehog*/*smoothened* pathway of signal transduction, first identified in Drosophila, are conserved in diverse living organisms from Drosophila to Human. However the pathway gets more complex in more advanced organisms (e.g. presence in human of more than one genes that display significant similarity to the single *patched* gene of Drosophila). Inactivating mutations of the *patched* have been found to cause constitutive (ligand-free) signalling through the *hedgehog*/*smoothened* pathway. The *hodgehog*/*smoothened* pathway overactivity, resulting from mutations of the *patched* and/or further downstream pathway elements, is found in all BCC's. The nevoid basal cell carcinoma syndrome (NBCCS) results from patched haploinsufficiency. Patients with the NBCCS, because of an already mutant *patched* in all cells, develop multiple BCC's as they grow older.

Cyclopamine, a steroid alkaloid, has the chemical formula shown below.

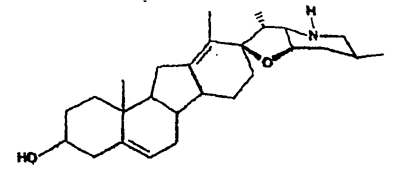

It is found naturally in the lily *Veratrum californicum* and can be obtained by purification from this and other sources. Inhibition of the *hedgehog*/*smoothened* pathway by cyclopamine has been found in chicken embryos and in cultured cells of mouse.

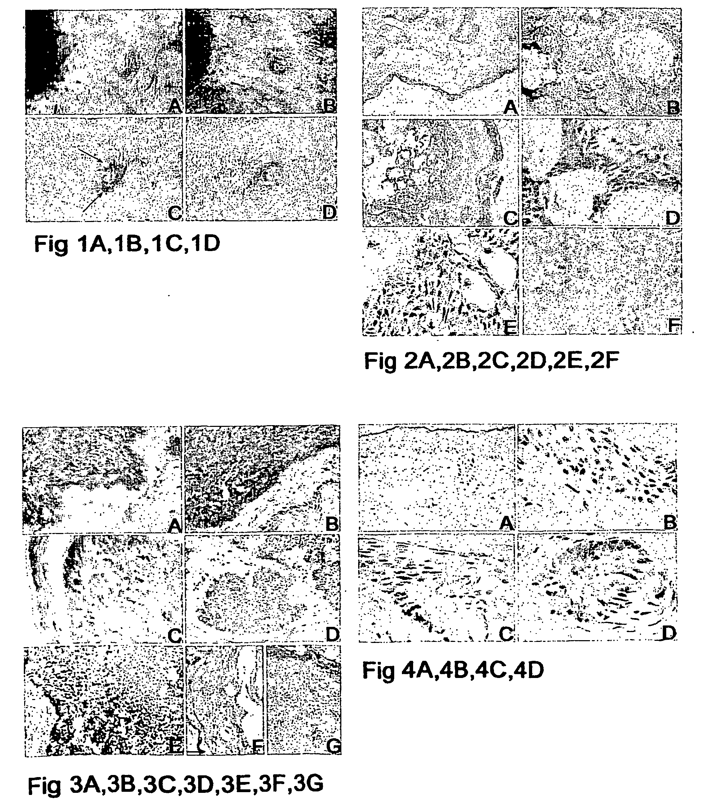

For topical applications, cyclopamine can be dissolved in ethanol or another suitable solvent and mixed with a suitable base cream, ointment or gel. Cydopamine may also be entrapped in hydrogels or in other pharmaceutical forms enabling controlled release and may be adsorbed onto dermal.patches. The effects shown in figures Fig 1A to Fig 1D, Fig 2A to Fig 2F, Fig 3A to Fig 3G and Fig 4A to Fig 4D have been obtained by a cream preparation obtained by mixing a solution of cyclopamine in ethanol with a base cream so as to get a final concentration of 18 mM cyclopamine in cream. The base cream used is made predominantly of heavy paraffin oil(10% w/w), vaseline (10% w/w), stearyl alcohol (8% w/w), polyoxylsteareth-40 (3% w/w) and water (68% w/w) but another suitably formulated base cream is also possible. Optimal concentration of cyclopamine in a pharmaceutical form as well as the optimal dosing and application schedules can obviously be affected by such factors as the particular pharmaceutical form, the localisation and characteristics of the skin containing the tumor (e.g. thickness of the epidermis) and the tumor size; however these can be determined by following well known published optimisation methods. The dosing and the application schedules followed for the tumors shown in Fig 1A (BCC on the nasolabial fold, ~ 4x5 mm on surface) and Fig 1C (BCC on the forehead, - 4x4 mm on surface) are as follows: 10 ± 2 µl cream applied directly onto the BCC's with the aid of a steel spatula four times per day starting ~ 9.00 a.m. with ~ 3½ hours in between. Night-time applications, avoided in this schedule because of possible loss of cream from the patient skin to linens during sleep, can be performed by suitable dermal patches. Preservation of the undifferentiated cells in the normal epidermis and in hair follicles following exposure to cyclopamine, as described in this invention, provide information about the tolerable doses in other possible modes of administration as well; e.g. direct intratumoral injection of an aqueous solution or systemic administration of the same or of cydopamine entrapped in liposomes.
Fig 1A, Fig 1B, Fig 1C and Fig 1D show rapid clinical regressions of the BCC's following exposure to cyclopamine. Besides the visual disappearence of several tumor areas within less than a week of cyclopamine exposure, there is a loss in the typically translucent appearance of the BCC's as seen by the comparison of Fig 1B to Fig 1A and of Fig 1D to Fig 1C.
Fig 2A to Fig 2F show microscopic appearences of the tumor areas subjected to surgical excisions together with a margin of normal tissue on the fifth and sixth days of cyclopamine applications when the BCC's had lost most of their pre-treatment areas but still possessed few regions that, although markedly decreased in height, had not yet completely disappeared and therefore had residual *tumor* cells for microscopic analyses.
Fig 2A and Fig 2B show, on tissue sections, the skin areas corresponding to the visually disappeared tumor nodules. The tumors are seen to have disappeared to leave behind large cystic structures containing little material inside and no detectable tumor cells.
Fig 2C shows microscopic appearance of a skin area that contained still visible BCC in vivo. These regions are seen to contain residual BCC's displaying large cysts in the tumor center and smaller cystic structures of various sizes located among the residual BCC cells towards the periphery.
Fig 2D and Fig 2E show 1000X magnified appearances from the interior and palisading peripheral regions of these residual BCC's and show the presence of massive apoptotic activity among the residual BCC cells regardless of the tumor region. These high magnifications show greatly increased frequency of the BCC cells displaying apoptotic morphology and formation of the cystic structures by the apoptotic removal of cells as exemplified on Fig 2D by the imminent joining together of the three smaller cysts into a larger one upon removal of the apoptotic septal cells.
Fig 2F shows that the BCC's treated with the placebo cream (i.e. the cream preparation identical to the cyclopamine cream except for the absence of cyclopamine in placebo) show, by contrast, the typical neoplastic BCC cells and no detectable apoptotic activity.

Cells undergoing apoptosis are known to be removed by macrophages and by nearby cells in normal tissues and the quantitation of apoptotic activity by morphological criteria on hematoxylene-eosine stained sections is known to provide an underestimate. Despite these, the quantitative data shown in Table I show greatly increased apoptotic activity caused by cyclopamine among the residual BCC cells.

The loss of translucency in the cyclopamine-treated BCC's raises the intriguing possibility of differentiation of BCC's under the influence of cyclopamine. This possibility, which can be tested by immunohistochemical analyses of the BCC's, is found to be the case in this invention. In normal epidermis, differentiation of basal layer cells to the upper layer cells is accompanied by a loss of labelling with the monoclonal antibody Ber-Ep4. Ber-Ep4 labels also the BCC cells and is a known marker for these neoplasms. Fig 3A, Fig 3B and the quantitative data on Table I show that, while Ber-Ep4 strongly labels all peripheral palisading cells and over 90% of the interior cells of the placebo-treated BCC's, none of the residual peripheral or interior cells of the cyclopamine-treated BCC's are labelled by Ber-Ep4. Differentiation of the BCC's under the influence of cyclopamine, hitherto unknown by any other means and highly unusual because of achievement of it in vivo and in all cells by immunohistochemical criteria, has independent value in the treatment of cancer.

Another differentiation marker, *Ulex Europaeus* lectin type 1, normally does not label the BCC's or the basal layer cells of normal epidermis but labels the differentiated upper layer cells. Fig 3C, showing the heterogenous labelling of the residual cells of cyclopamine-treated BCC's with this lectin, shows differentiation of some of the BCC cells beyond the differentiation step detected by Ber-Ep4 all the way to the step detected by *Ulex Europaeus* lectin type 1.

The p53 is a master regulator of the cellular response to DNA-damage. Amount of this protein is known to increase in the cell nucleus following exposure of cells to genotoxic agents. When the DNA-damage is increased beyond a threshold, p53 serves for the apoptotic death of cells. Radiation therapy of cancer and the genotoxic cancer chemotherapeutics that are currently common, act largely by this mechanism, i.e. by causation of apoptosis secondary to the damaging of DNA. The monoclonal antibody DO-7 can bind both normal and missense mutant (i.e., nonfunctional) forms of p53 and is known to be capable of detecting the increase of p53 in the cells following exposure to DNA-damaging agents.

Fig 3D, Fig 3E and the quantitative data in Table I show that both the DO-7 labelling intensity and the frequency of labelled cells are markedly decreased in cyclopamine-treated BCC's in comparison to the placebo-treated BCC's. Thus cyclopamine causes, not an increase, but rather a decrease of p53 in the nuclei of cyclopamine-treated BCC cells. Since expression of p53 is known to decrease in epidermal cells upon differentiation, the decreased DO-7 labelling of the cyclopamine-treated BCC's is likely to be secondary to the cyclopamine-induced differentiation of the BCC cells. In any case, massive apoptotic activity in the cyclopamine-treated BCC's despite markedly decreased p53 expression means that the cyclopamine-induced apoptosis of these tumor cells is by a non-genotoxic mechanism.

Arrest of the proliferation of BCC's is known to be associated with their retraction from stroma. Although retraction from stroma can also be caused artefactually by improper fixation and processing of the tissues, adherence to published technical details ensures avoidance of such artefacts. As shown in Fig 3F and Fig 3G, cyclopamine-treated, but not placebo-treated, BCC's are consistently retracted from stroma. Exposure of BCC's to cyclopamine thus appears to be associated also with an arrest of proliferation.

Fig 4A to Fig 4D show Ber-Ep4 labelling of the normal skin tissue found on and around the cyclopamine-treated BCC's. Different epidermal areas that were treated with cyclopamine are seen in Fig 4A, Fig 4B and Fig 4C to display normal pattern of labelling with Ber-Ep4, i.e. labelling of the basal layer cells. Similarly Fig 4D shows normal Ber-Ep4 labelling of a hair follicle exposed to cyclopamine. Thus the undifferentiated cells of normal epidermis and of hair follicles are preserved despite being exposed to the same schedule and doses of cyclopamine as the BCC's.

Causation of highly efficient differentiation and apoptosis of the tumor cells in vivo by cyclopamine at doses that preserve the undifferentiated tissue cells are hitherto unknown achievements that, together with the non-genotoxic mode of action of cyclopamine, support the use of cyclopamine not only on BCC's but also on those internal tumors that utilize the *hedgehog*/*smoothened* pathway for proliferation and for prevention of apoptosis and/or differentiation.

### Description Of The Figures

Fig 1A, 1B, 1C, 1D: Rapid regressions of the cyclopamine-treated BCC's as indicated by disappeared tumor regions (exemplified by arrows), markedly decreased height from skin surface and by a loss of translucency in less than a week. 1A : BCC, located on left nasolabial fold, prior to treatment. 1B : Same BCC on the fifth day of topical cyclopamine treatment. 1C: BCC, located on forehead, prior to tretment. 1D : Same BCC on the sixth day of topical cyclopamine treatment.

Fig 2A, 2B, 2C, 2D, 2E, 2F: Microscopic appearences of the cyclopamine- and placebo-treated BCC's, showing the cyclopamine-induced massive apoptotic death and removal of the tumor cells and the disappearence of tumor nodules to leave behind cystic spaces with no tumor cells. Skin areas corresponding to the pre-treatment positions of the BCC's were exdsed surgically on the fifth and sixth days of cyclopamine exposure with a margin of normal tissue and subjected to conventional fixation, sectioning and hematoxylene-eosine staining for microscopic analyses. 2A : Large cyst in the dermis corresponding to the position of a disappeared tumor nodule showing no residual tumor cells. 2B: Similar cysts in another dermal area that contained BCC prior to, but not after, treatment with cydopamine. 2C : Low power view of an area of the BCC shown on Fig 1 D showing residual cells and formation of a large cyst by the joining together of the numerous smaller cysts in between these cells. 2D : High power view from an interior region of the same residual BCC as in Fig 2C showing greatly increased frequency of the apoptotic cells and the formation as well as enlargement of the cysts by the apoptotic removal of the BCC cells. 2E : High power view from from a peripheral region of the same residual BCC as in Fig 2C also showing greatly increased frequency of the apoptotic cells and the formation of cysts by the apoptotic removal of BCC cells. 2F : High power view from an internal area of a placebo-treated BCC showing typical neoplastic cells of this tumor and the absence of apoptosis. Original magnifications are 100X for 2A, 2B, 2C and 1000X for 2D, 2E, 2F.

Fig 3A, 3B, 3C, 3D, 3E, 3F, 3G : Immunohistochemical analyses of the cyclopamine- and placebo-treated BCC's showing differentiation of all residual BCC cells under the influence of cyclopamine and the decrease of p53 expression in BCC's following exposure to cyclopamine. 3A and 3B : Absence of staining with the monoclonal antibody Ber-Ep4 in all residual cells of cyclopamine-treated BCC (3A) contrasted with the strong staining in placebo-treated BCC (3B) showing that all residual cells in the cyclopamine-treated BCC's are differentiated to or beyond a step detected by Ber-Ep4. Ber-Ep4 is a known differentiation marker that stains the BCC cells as well as the undifferentiated cells of the normal epidermis basal layer and of hair follicles but not the differentiated upper layer cells of normal epidermis. 3C: Heterogenous labelling of the residual cells of a cyclopamine-treated BCC with the *Ulex* Europaeus lectin type 1 showing differentiation of some of the BCC cells all the way to the step detected by this lectin which normally does not label the BCC's or the basal layer cells of the normal epidermis but labels the differentiated upper layer cells. 3D and 3E: Decreased expression of p53 as detected by the monoclonal antibody DO-7 in cyclopamine-treated BCC's (3D) in comparison to the placebo-treated BCC's (3E). Expression of p53 is known to decrease upon differentiation of the epidermal basal cells and upon differentiation of cultured keratinocytes. It is also well known that the amount of p53, detectable by DO-7, increases in cells when they are exposed to DNA-damaging agents. 3F and 3G : Consistent retraction of BCC's from stroma, which is a feature known to be associated with the arrest of tumor cell proliferation, seen in cydopamine-treated (3F, arrow shows the retraction space) but not in placebo-treated (3G) tumors (difference of the cyclopamine- and placebo-treated BCC's in terms of retraction from stroma is seen also in 3D, 2C vs 3B, 3E). Original magnifications are 400X for 3A, 3B, 3D, 3E, 1000X for 3C and 100X for 3F, 3G. All immunohistochemical labellings are with peroxidase-conjugated streptavidin binding to biotinylated secondary antibody; labelling is indicated by the brown-colored staining. Sections shown in 3F and 3G are stained with Periodic Acid-Schiff and Alcian blue.

Fig4A, 4B, 4C, 4D: Normal pattern of labelling of the cyclopamine-treated normal skin with Ber-Ep4 showing that the undifferentiated cells of normal epidermis and of hair follicles are preserved despite being exposed to the same schedule and doses of cyclopamine as the BCC's. 4A : Ber-Ep4 labelling of the basal layer cells of the epidermis treated with cyclopamine. 4B and 4C : Higher power views from different areas of cyclopamine-treated epidermis showing Ber-Ep4 labelling of the basal cells. 4D : High power view of a hair follicle treated with cyclopamine yet showing normal labelling with Ber-Ep4. Original magnification is 400X for 4A and 1000X for 4B, 4C, 4D. Immunohistochemical detection procedure is the same as in Fig 3A, 3B; labelling is indicated by brown coloring.

**Table 1:**

| Induction of the Differentiation and Apoptosis of Basal Cell Carcinoma Cells by Topical Cyclopamine | | | | |
|---|---|---|---|---|
| | Peripheral Palisading Cells of the BCC's Treated With | | Non-Palisading Cells of the BCC's Treated With | |
| | Placebo | Cyclopamine | Placebo | Cyclopamine |
| % Of Cells Showing ≥ 2 Morphological Signs Of Apoptosis On H&E Stained Tissue Sections | 0 ± 0 | 20 ± 8 | 0.2 ± 0.4 | 18 ± 11 |
| | | | | |
| % Of Cells Labelled With Ber-Ep4 | 100 ± 0 | 0 ± 0 | 91 ± 8 | 0 ± 0 |
| | | | | |
| % Of Cells Labelled With DO-7 | 58 ± 27 | 16 ± 11 | 67 ± 22 | 5 ± 3 |
| Means ± standart deviations from at least 16 randomly selected high-power (1000 X) fields of the tissue sections of each tumor group are shown. p < 0.001 for the placebo vs cyclopamine-treated tumors for all the parameters, both for the palisading peripheral and the non-palisading (interior) tumor areas. | | | | |

## Claims

1. Use of cyclopamine or a pharmaceutically acceptable salt thereof for the manufacture of a medicament for obtaining decreased size or disappearance of a tumor employing hedgehog/smoothened signalling for the prevention of apoptosis and/or for the prevention of differentiation of tumor cells, wherein said medicament is to be administered in an amount which induces differentation and apoptosis of said tumor cells.

2. Use according to claim 1, for treatment of basal cell carcinoma.

3. Use according to any one of claims 1 or 2, **characterized in that** said medicament is formulated for topical, non-topical or systemic administration.

4. Use according to claim 3, **characterized in that** for non-topical administration cyclopamine or a pharmaceutically acceptable salt thereof is in the form of an aqueous solution, or is entrapped in liposomes.

5. Use according to any one of claims 1 to 4, **characterized in that** said medicament is a pharmaceutical form enabling controlled release.

6. Use according to any one of claims 1 to 5, **characterized in that** said medicament is for administration by direct intratumoral injection.

7. Use according to any one of claims 1 to 5, **characterized in that** said medicament is adsorbed onto a dermal patch.

8. Use according to any one of claims 1 to 3, **characterized in that** said medicament is manufactured in the form of a cream, ointment, gel or hydrogel.

9. Use according to claim 8, **characterized in that** said medicament is manufactured in the form of a cream comprising 18 mM of cyclopamine or a pharmaceutically acceptable salt thereof.

## Patentansprüche

1. Verwendung von Cyclopamin oder eines pharmazeutisch akzeptablen Salzes davon für die Herstellung eines Medikaments zum Erreichen verringerter Größe oder des Verschwindens eines Tumors welcher dem *hedgehog*/*smoothened* Signalweg zur Verhinderung von Apoptose und/oder zur Verhinderung von Differenzierung von Tumorzellen verwendet, wobei das Medikament in einer Menge zu verabreichen ist welche Differenzierung und Apoptose der Tumorzellen induziert.

2. Verwendung nach Anspruch 1, zur Behandlung von Basalzellkarzinomen.

3. Verwendung nach einem der Ansprüche 1 oder 2,
**dadurch gekennzeichnet, dass** das Medikament für topische, nicht-topische oder systemische Verabreichung formuliert ist.

4. Verwendung nach Anspruch 3,
**dadurch gekennzeichnet, dass** für die nicht-topische Verabreichung das Cyclopamin oder ein pharmazeutisch akzeptables Salz davon in Form einer wässrigen Lösung vorliegt oder in Liposomen eingebaut ist.

5. Verwendung nach einem der Ansprüche 1 bis 4,
**dadurch gekennzeichnet, dass** das Medikament eine pharmazeutische Form ist, welche eine kontrollierte Freisetzung ermöglicht.

6. Verfahren nach einem der Ansprüche 1 bis 5,
**dadurch gekennzeichnet, dass** das Medikament für die Verabreichung mittels direkter intratumoraler Injektion vorliegt.

7. Verwendung nach einem der Ansprüche 1 bis 5,
**dadurch gekennzeichnet, dass** das Medikament auf einem Hautpflaster adsorbiert ist.

8. Verwendung nach einem der Ansprüche 1 bis 3,
**dadurch gekennzeichnet, dass** das Medikament in Form einer Creme, Salbe, eines Gels oder Hydrogels hergestellt ist.

9. Verfahren nach Anspruch 8,
**dadurch gekennzeichnet, dass** das Medikament in Form einer Creme umfassend 18 mM Cyclopamin oder eines pharmazeutisch akzeptablen Salzes davon hergestellt ist.

## Revendications

1. Utilisation de la cyclopamine ou d'un sel pharmaceutiquement acceptable de celle-ci pour la fabrication d'un médicament afin de faire diminuer ou de faire disparaître une tumeur, avec l'utilisation de la voie de signalisation Hedgehog/Smoothened afin d'empêcher l'apoptose et/ou pour empêcher la différenciation des cellules tumorales, dans laquelle ledit médicament doit être administré avec une quantité qui provoque la différenciation et l'apoptose desdites cellules tumorales.

2. Utilisation selon la revendication 1, pour le traitement du carcinome des cellules basales.

3. Utilisation selon l'une des revendications 1 ou 2, **caractérisée en ce que** ledit médicament est formulé pour une administration topique, non topique ou systémique.

4. Utilisation selon la revendication 3, **caractérisée en ce que**, pour l'administration non topique, la cyclopamine ou un sel pharmaceutiquement acceptable de celle-ci se présente sous la forme d'une solution aqueuse ou est piégée dans des liposomes.

5. Utilisation selon l'une des revendications 1 à 4, **caractérisée en ce que** ledit médicament est une forme pharmaceutique permettant une libération contrôlée.

6. Utilisation selon l'une des revendications 1 à 5, **caractérisée en ce que** ledit médicament est prévu pour une administration par injection intra-tumorale directe.

7. Utilisation selon l'une des revendications 1 à 5, **caractérisée en ce que** ledit médicament est adsorbé sur un patch dermique.

8. Utilisation selon l'une des revendications 1 à 3, **caractérisée en ce que** ledit médicament est fabriqué sous la forme d'une crème, d'un onguent, de gel ou d'un hydrogel.

9. Utilisation selon la revendication 8, **caractérisée en ce que** ledit médicament est fabriqué sous la forme d'une crème comprenant 18 mM de cyclopamine ou d'un sel pharmaceutiquement acceptable de celle-ci.
